# FASCICULE DE BREVET EUROPEEN

(11) **EP 2 004 183 B1**
(45) Date de publication et mention de la délivrance du brevet: **26.06.2013**
(21) Numéro de dépôt: 07727980.0
(22) Date de dépôt: 11.04.2007
(51) Int. Cl.: A61K 45/06

(54) **ASSOCIATION DU MAZINDOL DANS LE TRAITEMENT DU DEFICIT DE L'ATTENTION/HYPERACTIVITE**
MAZINDOL-KOMBINATION BEI DER BEHANDLUNG VON AUFMERKSAMKEITSDEFIZIT/HYPERAKTIVITÄT
MAZINDOL COMBINATION IN THE TREATMENT OF ATTENTION DEFICIT/HYPERACTIVITY

(30) Priorité: 11.04.2006 FR 0603197
(43) Date de publication de la demande: 24.12.2008
(60) Demande divisionnaire: 12189703.7
(73) Titulaire: Assistance Publique - Hôpitaux de Paris, 75004 Paris (FR)
(72) Inventeur: KONOFAL, Eric, 60300 Senlis (FR)
(74) Mandataire: Regimbeau
(86) Numéro de dépôt international: PCT/EP2007/053512
(87) Numéro de publication internationale: WO 2007/116076

(56) Documents cités:
- WO-A-91/11184
- WO-A-93/21917
- WO-A-02/053104
- WO-A-2004/091546
- WO-A-2004/105744
- GB-A- 2 144 410
- DATABASE BIOSIS [Online] BIOSCIENCES INFORMATION SERVICE, PHILADELPHIA, PA, US; 2002, VAZQUEZ-ALVAREZ A ET AL: "MAZINDOL EFFECTS ON LEAD - INDUCED LOCOMOTOR HYPERACTIVITY IN MICE." XP002395485 Database accession no. PREV200300327254 & SOCIETY FOR NEUROSCIENCE ABSTRACT VIEWER AND ITINERARY PLANNER, vol. 2002, 2002, pages Abstract No. 804.6 URL-http://sf, 32ND ANNUAL MEETING OF THE SOCIETY FOR NEUROSCIENCE; ORLANDO, FLORIDA, USA; NOVEMBER 02-07, 2002
- WALTHER B W: "TREATING RESTLESS LEGS SYNDROME: CURRENT PATHOPHYSIOLOGICAL CONCEPTS AND CLINICAL TRIALS" EXPERT OPINION ON INVESTIGATIONAL DRUGS, ASHLEY PUBLICATIONS LTD., LONDON, GB, vol. 11, no. 4, avril 2002 (2002-04), pages 501-514, XP009024933 ISSN: 1354-3784
- ZHOU J: "Norepinephrine transporter inhibitors and their therapeutic potential" DRUGS OF THE FUTURE 2004 SPAIN, vol. 29, no. 12, 2004, pages 1235-1244, XP002395478 ISSN: 0377-8282

## Description

La présente invention concerne le domaine de la santé humaine et plus particulièrement le traitement du trouble déficit de l'attention/hyper-activité (TDAH) par le mazindol. Ce dernier peut être administré monothérapie ou en association avec un ou plusieurs composés, y compris des psychostimulants, pour l'indication de TDAH et des symptômes associés ou comorbides.

Le TDAH est un trouble comportemental qui constitue un des motifs les plus fréquemment rencontrés en psychopathologie de l'enfant et de l'adolescent. Sa prévalence est estimée entre 2 à 5% dans la population générale des enfants en âge scolaire.

Sur le plan clinique, ce trouble associe une inattention, une impulsivité et une hyperactivité motrice inadaptée à l'environnement de l'enfant. Mal organisés et étourdis, ces enfants finissent parfois par ne plus suivre en classe. L'agitation motrice excessive, incompatible avec les relations sociales et pouvant parfois même conduire à une déscolarisation prématurée, est probablement le symptôme qui amènera les parents à consulter un spécialiste.

Les substances éveillantes utilisées, et couramment admises dans le traitement pharmacologique du TDAH, notamment chez l'enfant appartiennent à plusieurs classes pharmacologiques : les psychostimulants (amphétamine, méthylphénidate, bupropion), les eugrégoriques (modafinil, adrafinil), et les inhibiteurs de la mono-amine oxydase B (sélégiline).

Les plus utilisés et les mieux connus sont :
- le méthylphénidate (MPH) est le traitement de référence du TDAH chez l'enfant, l'adolescent et l'adulte. C'est surtout un psychostimulant connue pour ses propriétés éveillantes. Outre son action stimulante dopaminergique, sur la libération de la noradrénaline et de la dopamine, par inhibition de la recapture, le MPH est dépourvu d'effet sur les récepteurs alpha-1 noradrénergiques postsynaptiques (modification de la sensibilité).
- L'amphétamine (d/l-amphétamine) a une action sur la libération extra-vésiculaire de la noradrénaline et de la dopamine et donc inhibe toute forme de stockage. En raison d'un mésusage potentiel, et des effets indésirables périphériques (tachycardie, HTA, agitation, insomnie) sa médication reste très limitée, et non autorisée dans la plupart des pays de l'Europe.
- Le modafinil, dont l'autorisation pour le traitement du TDAH chez l'enfant vient d'être accordée récemment aux Etats-Unis (2005), est un médicament éveillant (eugrégorique) dont le mécanisme d'action, complexe, est imparfaitement connu. Contrairement au MPH et aux amphétamines, le modafinil n'induit pas de dépendance ni d'accoutumance. Sa prescription est aujourd'hui limitée en France au traitement de la narcolepsie et de l'hypersomnie idiopathique.
- L'atomoxétine, inhibiteur sélectif de la recapture de la noradrénaline, et stimulant dopaminergique (par inhibition de la recapture au niveau du cortex pré-frontal), a montré une efficacité et une bonne tolérance dans le TDAH de l'enfant et de l'adulte (Spencer et al., 1998 ; Popper, 2000 ; Biederman et al., 2002). Son autorisation de mise sur le marché aux Etats-Unis est récente (FDA, novembre, 2002).
- Autres : le bupropion, la caféine, la sélégiline ...
   Le bupropion, inhibiteur de recapture des catécholamines, antidépresseur est également un compétiteur potentiel dans le traitement du TDAH.
   La sélégiline, inhibiteur de recapture de la mono-oxidase, possède également des propriétés pharmacologiques proche de celles des amphétamines. Son action éveillante dans le traitement du TDAH est connue, son intérêt dans cet usage possible.

Ainsi, l'amélioration de l'hyperactivité motrice par les psychostimulants dopaminergiques est souvent très significative mais néanmoins insuffisante.

En effet, les substances éveillantes utilisées ou qui pourraient être utilisées dans le traitement du TDAH, notamment les psychostimulants tels que le méthylphénidate ou les amphéntamines, ont souvent des durée de demi-vie plasmatique courte ce qui implique l'apparition d'effets « on-off », c'est-à-dire un effet de sevrage qui s'accompagne d'un effet « rebond de symptômes » après quelques heures et responsable d'une recrudescence des symptômes en dernière partie de nuit, et nuisible à la qualité de l'endormissement.

De plus, certaines de ces substances sont métabolisées dans l'organisme et présentent donc un risque toxique pour le patient.

En outre certains des médicaments administrés dans le traitement du TDAH ne sont pas adaptés à une administration particulière aux enfants, notamment à cause d'une taille trop importante des comprimés ou une administration pluri-journalière des médicaments.

De plus, certains symptômes particuliers tels que les insomnies, les difficultés d'endormissement, les réveils au cours de la nuit, éventuellement dus à une agitation motrice nocturne excessive, ainsi que les troubles attentionnels, tels que l'inattention, l'impatience et l'impulsivité semblent échapper à toute forme de traitement [Chervin et al., Associations between symptoms of inattention, hyperactivity, restless legs, and periodic leg movements. Sleep 2002 15;25(2):213-8; Gruber et al., instability of sleep patterns in children with attention-deficit/hyperactivity disorder. J Am Acad Child Adolesc Psychiatry. 2000;39(4):495-501].

Il existe donc un réel besoin de développer de nouveaux traitements du TDAH qui permettent d'obtenir des résultats supérieurs à ceux obtenus avec les traitements actuels à base de psychostimulants et notamment de pouvoir traiter les symptômes qui échappent aux traitements actuels, sans effet de sevrage ni rebond des symptômes et présentant un risque toxique limité. Des traitements destinés plus particulièrement aux enfants sont avantageusement recherchés. C'est le but de la présente invention.

La demande W02004/105744 concerne l'utilisation du fer pour la préparation d'un médicament destiné au traitement préventif et/ou curatif du TDAH ou d'au moins un de ses symptômes.

Le document Vazquez-Alvarez et al. (2002) "Mazindol effects on lead-induced locomotor hyperactivity in mice" (32nd annual meeting of the society for neuroscience) résume une présentation concernant le traitement de l'hyperkinésie induite par une intoxication au plomb chez des souris par le mazindol.

La demande W02004/091546 concerne le traitement du trouble du déficit de l'attention (TDA), du TDAH, de la narcolepsie, de l'addiction à la cocaïne, de la suppression de l'appétit et de la dépression par une combinaison d'un stimulant du système nerveux central et d'un antagoniste d'opioïde.

De manière tout a fait fortuite, des études ont maintenant montré que le mazindol pouvait être utilisé pour le traitement préventif et curatif du TDAH avec des résultats significatifs, sans les inconvénients précédemment cités des autres substances, en particulier de certains psychostimulants.

Le mazindol présente la formule chimique suivante :
5-(4-chlorophényl)-2,5-dihydro-3H-imidazo[2,1-a]isoindol-5-ol

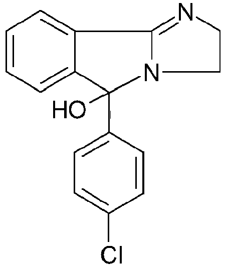

Le mazindol est considéré, dans les classifications médicamenteuses actuelles, comme un médicament psychoanaleptique et anorexigène, mais aussi pourvoyeur d'éveil, actuellement non autorisé en France, ou seulement autorisé par ATU (autorisation temporaire d'utilisation) dans l'obésité et le narcolepsie. C'est un composé chimique intéressant pour la prise en charge des dysfonctionnements des mécanismes de l'éveil.

L'action pharmacologique essentielle du mazindol, dans l'ensemble des espèces étudiées, chez l'animal sain comme chez l'homme, est hypothalamique, sur les centres dopaminergiques régulateurs de l'appétit (Hadler, 1972). Son métabolisme principal est urinaire (3/4 urine, ¼ fèces).

Le mazindol est un composé non-amphétaminique en raison de sa structure chimique tricyclique. Il offre un profil pharmacologique très voisin de celui des amphétamines sous en reproduire les effets secondaires. Ainsi, contrairement aux molécules amphétaminiques, le mazindol n'accroît l'activité motrice qu'en relation avec l'allongement de la durée de l'éveil et n'induit pas de stéréotypies ni de modifications cardio-vasculaires (Hadler, 1972).

De plus, dans les études de toxicologie animale, le potentiel toxique du mazindol s'est avéré très faible. En effet, le mazindol présente un risque toxique limité car les métabolites du mazindol, sont excrétés dans l'urine. En particulier, il n'a été observé :
- aucun effet carcinogène ;
- aucun effet mutagène ;
- aucun effet en toxicologie de la reproduction.

En outre, le Mazindol possède un temps de demi-vie plasmatique long, supérieur à une journée, ce qui évite l'apparition d'effets sevrage, et donc d'un effet « rebond de symptômes » en fin de journée.

En effet, après administration orale unique ou répétée, le mazindol est absorbé avec un tmax de 2-4 heures. La prise concomitante d'alimentation est susceptible de retarder (d'environ 1 h) l'absorption mais ne modifie pas la quantité totale absorbée. Le temps de demi-vie plasmatique est atteint après 33-55 heures.

La pharmacocinétique est linéraire (indépendante de la dose) pour des doses comprises entre 1 mg/j et 4 mg/j, et 75 % de la dose reste est encore « plasmatiquement » efficace 24 heures après la prise.

De plus, les comprimés de mazindol (Teronac^{®}) sont petits, et ne posent donc pas de problème d'administration. Pour des raisons pharmacocinétiques, déjà citées, le mazindol n'est administré qu'une fois par jour, ce qui limite les contraintes pour le patient, et notamment dans le cas particulier des enfants pour l'école qui est souvent sollicitée pour la dispensation du midi.

Le mazindol a fait l'objet depuis plus de 30 ans de nombreuses études contrôlées en double aveugle contre placebo sur le traitement de l'obésité chez l'adulte. Les études dans le traitement de la narcolepsie, de l'hypersomnie sont limitées. Par contre, la sécurité d'emploi à court, moyen et long terme dans le traitement de la somnolence diurne excessive associée à la narcolepsie et à l'hypersomnie idiopathique est relativement bien documenté pour l'époque (Shindler, et al., 1985).

Le mazindol est devenu dans ces deux affections (narcolepsie et hypersomnie) un traitement en ATU (Autorisation Temporaire d'Utilisation) de troisième intention qui permet d'améliorer significativement la qualité de vie des patients présentant un trouble du maintient de l'éveil.

La présente invention a donc pour objet l'utilisation du mazindol pour la préparation d'un médicament destiné au traitement préventif et/ou curatif du trouble du déficit de l'attention/hyperactivité (TDAH) selon les critères du Manuel Diagnostique et Statistique des troubles mentaux, 4ème édition (DSM IV), chez un patient nécessitant un tel traitement, la posologie correspondant à une prise journalière de mazindol entre 1 et 2 mg.

Dans le cadre de la présente invention, le diagnostic de Trouble Déficit de l'Attention/Hyperactivité (TDAH) est fondé selon les caractéristiques cliniques définies par la classification internationale, la Manuel Diagnostique et Statistique des troubles mentaux, DSM/IV (Diagnostic and Statistical Manual of mental disorders, 4^{ème} ed., 1994).

Les critères du DSM-IV incluent trois dimensions (inattention, impulsivité et hyperactivité), une efficience intellectuelle normale (QI>80, d'âge compris entre 5 et 12 ans), et présentant une carence martiale isolée mais non anémié, c'est-à-dire présentant un taux d'hémoglobine normal. Par l'expression « carence martial », on entend une hypoferritinémie sans modification significative de la concentration sérique en récepteurs solubles de la transferrine.

Le patient selon l'invention est choisi parmi un nouveau-né, un enfant, un adolescent, un adulte. Selon un mode préféré de réalisation, il s'agit d'un enfant d'âge environ de 5 à 12 ans, et/ou un adolescent. Le patient selon l'invention est avantageusement affecté d'une carence martiale mais non anémié. La carence en ferritine peut être mesuré dans le sérum, mais également dans tout autres liquides biologiques tels que le liquide céphalo-rachidien.

Une carence en ferritine correspond à une concentration sérique en ferritine du patient adulte inférieure à environ 50µg/litre. Cette hypoferritinémie peut atteindre des concentrations en ferritine inférieures à environ 40µg/l, voire inférieures à environ 35µg/l, inférireures à 30 µg/l, inférieures à 20µg/l, inférieures à 15µg/l, voire même inférieures à environ 10µg/l. Les techniques de dosage de la ferritine sérique sont bien connues de l'homme du métier. On peut citer la méthode immunoenzymatique (Kit IMX ferritine, Abott Laboratories).

Le patient selon l'invention présente en outre une concentration sérique normale de récepteurs solubles à la transferrine. La transferrine est impliquée dans l'acquisition du fer par les cellules de l'organisme ; cette acquisition est contrôlée par le nombre de récepteurs à la transferrine existant à la surface cellulaire. La concentration de ces récepteurs peut être évalués par des techniques connues de l'homme du métier telles que la néphélémétrie (Ruivard et al., 2000 Rev. Méd. Interne 21 : 837-843). Une fourchette de concentration normale des récepteurs solubles à la transferrine est de 2,0-4,50 mg/l pour les hommes et de 1,80-4,70 mg/l pour les femmes (voir Kit RsTF Réf.2148315 de Roche).

Les composés ou compositions selon l'invention peuvent être administrés de différentes manières et sous différentes formes. Ainsi, ils peuvent être administrés de manière systémique, par voie orale, par voie anale ou par voie parentale notamment par inhalation ou par injection, comme par exemple par voie intraveineuse, intra-musculaire, sous-cutanée, trans-dermique, et intra-artérielle. De préférence, il s'agit de la voie orale.

Pour les injections, les composés sont généralement conditionnés sous forme de suspensions liquides, qui peuvent être injectées au moyen de seringues ou de perfusions, par exemple. A cet égard, les composés sont généralement dissous dans des solutions salines, physiologiques, isotoniques, tamponnées, etc., compatibles avec un usage pharmaceutique et connue de l'homme du métier. Ainsi, les compositions peuvent contenir un ou plusieurs agents ou véhicules choisis parmi les dispersants, solubilisants, stabilisants, conservateurs, etc. Des agents ou véhicules utilisables dans des formulations liquides et/ou injectables sont notamment la méthylcellulose, l'hydroxyméthylcellulose, la carboxyméthylcellulose, le polysorbate 80, le mannitol, la gélatine, le lactose, des huiles végétales, l'acacia, etc.

Les composés peuvent également être administrés sous forme de gels, huiles, comprimés, suppositoires, poudres, gélules, capsules, aérosols, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

Par « symptôme du TDAH », on entend désigner notamment les troubles attentionnels tels que l'inattention, l'impulsivité, l'impatience, les troubles oppositionnels, mais également l'hypcractivité motrice diurne ou nocturne, le syndrome des jambes sans repos, et les insomnies.

Par insomnie on entend désigner :
a. l'insomnie par endormissement qui se caractérise par des difficultés à s'endormir ;
b. l'insomnie de maintenance qui se caractérise par une hyperactivité motrice nocturne et des réveils en cours de nuit, et ;
c. l'insomnie psychopathologique généralement chronique et généralement liée à une anxiété, au stress et à des épisodes dépressifs.

Selon un autre aspect de la présente invention, le mazindol est utilisé en association avec du fer comme produit de combinaison pour une utilisation simultanée, séparée ou étalée dans le temps.

Selon un mode préféré d'utilisation, le fer est utilisé en supplémentation chez le patient avant l'administration du mazindol.

Au sens de la présente invention, on entend par « fer », le fer sous la forme d'un atome de fer, de sel de fer, ou de fer organique, ou de toute formulation contenant du fer qui soit pharmaceutiquement acceptable. A titre de liste non exhaustive, le sel de fer pharmaceutiquement acceptable est sélectionné parmi les sels ferreux et les sels ferriques, de préférence parmi l'ammonium citrate ferrique, le pyrophosphate ferrique, le ferrocholinate, l'abscorbate ferreux, l'aspartate ferreux, le chlorure ferreux, le sulfate ferreux, la tartatre ferreux, le fumatre ferreux, le gluconate ferreux, le gluceptate ferreux, le sulfate de glycine ferreux, le lactate ferreux, l'oxalate ferreux, le succinate ferreux.

Selon un mode préféré de l'invention, le sel de fer est le sulfate ferreux, et de préférence du sulfate ferreux gastro-protégé.

Alternativement, le fer pharmaceutiquement acceptable est sous la forme de fer dextran, de fer sucrose, de fer poly-maltose, de fer sorbitol. Lorsque le fer est sous la forme de fer organique pharmaceutiquement acceptable, il s'agit de préférence de biglycinate de fer, de glycinate de fer ou de fer protéine succinylate.

Selon un mode préféré de réalisation, l'utilisation du mazindol éventuellement en association avec le fer selon l'invention, est réalisée en association avec au moins un composé sélectionné parmi les psychostimulants, comme produit de combinaison pour une utilisation simultanée, séparée ou échelonnée dans le temps.

Par composés psychostimulants, on entend désigner les inhibiteurs de la recapture de la dopamine et/ou de la noradrénaline et les agonistes des catécholamines. Parmi ceux-ci, il convient de citer à titre non exhaustif
1) les composés psychostimulants : le méthylphénidate (spécialité Ritaline, Concerta, Equasym), le modafinil (Sparlon, Modiodal, Provigil), l'atomoxétine (Strattera), et les amphétamines, telles que la d-amphétamine, la déxédrine, la dexamphétamine.
2) L-Dopa : Modopar, Sinemet
3) les agonistes sélectifs de la dopamine : pramipexole (Sifrol, Mirapex), ropinirole (Requip, Adartrel), lisuride, pergolide, cabergoline...

Le rôle du fer au niveau du système nerveux central est souvent rapporté en neurophysiopathologie fondamentale comme clinique. Une asthénie fonctionnelle, intellectuelle, un syndrome de fatigue chronique, ou à l'inverse une instabilité psychomotrice et une irritabilité peuvent être la conséquence d'une carence martiale (Lozoff, 1989 Adv Pediatr 1989 ; 6 :331-59). Le rôle du fer dans la physiopathologie de maladies neurologiques, et notamment dans la Maladie de Parkinson Idiopathique est connu depuis plus de trente ans. L'évidence d'une augmentation martiale notamment dans certaines structures cérébrales (p.e. noyau denté) dans des pathologies neurodégénératives rares (p.e. ataxie de Friedreich) est également connue. Plus récemment, le rôle des récepteurs de la transferrine dans certains processus neurophysiopathologiques vient d'être documenté (Marder F, et al. 1998 Neurology 50, 4 :1138-40). Une augmentation en nombre des récepteurs de la transferrine des cellules de l'endothélium des capillaires cérébraux pourrait être responsable de l'accumulation de ganglions de la base (globus pallidus, substantia nigra, noyau rouge, et noyau denté). Un dysfonctionnement des récepteurs de la transferrine par hyperplasie (augmentation du nombre des récepteurs) au niveau central expliquerait l'accumulation du fer dans certaines structures impliquées dans les phénomènes de neurodégénérescence. A contrario, une diminution de ces récepteurs contribuerait à protéger les noyaux centraux du phénomène. Dans l'hypothèse d'une diminution de la ferritine plasmatique dans la dans la physiopathologie du TDAH, une augmentation physiologique des récepteurs de la transferrine devrait se produire, comme selle se produit normalement en cas d'anémie, afin de ne pas mettre les structures cérébrales en carence martiale. Par contre, une absence de réponse (absence d'augmentation du nombre des récepteurs de la transferrine) conduirait une diminution martiale cérébrale et serait compatible avec un dysfonctionnement dopaminergique par baisse de sa synthèse et/ou de la stimulation des récepteurs dopaminergiques. La présente invention concerne donc également l'utilisation du mazindol, optionnellement en association avec du fer ou l'un de ses sels pharmaceutiquement acceptables, et/ou un psychostimulant pour le traitement préventif de patient nouveau-né, enfant, adolescent, jeune adulte amené à développer à l'âge adulte une pathologie neuro-dégénérative caractérisée en ce que ledit patient nouveau-né, enfant, adolescent, jeune adulte présente au moins les symptômes suivants :
- une carence en ferritine, de sorte que la concentration sérique en ferritine est inférieure à 50 µg/l ;
- une concentration sérique normale des récepteurs solubles à transferrine ;
- un trouble du déficit de l'attention/hyperactivité.

De préférence ledit patient est un enfant avec un QI>80, d'âge compris entre environ 5 et 12 ans et non anémié.

De préférence ladite pathologie neurodégénérative est la maladie de Parkinson, les ataxies cérébelleuses, l'ataxie de Friedrich, la maladie d'Alzheimer, la chorée de Huntington, la sclérose latérale amyotrophique. Plus particulièrement, il s'agit de la maladie de Parkinson.

En particulier, lorsque le mazindol est utilisé en association avec le sulfate ferreux, la quantité de sulfate ferreux administrée au patient de façon journalière est comprise entre 0,1 mg et 10 g, de préférence comprise entre 100 mg et 2 g par jour, de préférence environ 500 mg, en une ou plusieurs prises.

Plus particulièrement, selon la présente invention, les patients subissent une supplémentation en fer, en particulier en sulfate ferreux, pendant 12 semaines puis un traitement au mazindol pendant 12 semaines.

La posologie correspond à une prise journalière de mazindol comprise entre 1 et 2 mg (doses recommandées dans le traitement de la narcolepsie chez l'adulte).

Les critères d'évaluation de l'efficacité du traitement du trouble déficit de l'attention/hyperactivité par le mazindol de manière optionnelle en association avec le fer et/ou un psychostimulant dans le traitement du trouble déficit de l'attention/hyperactivité selon la présente invention sont la réduction (>30%) du score sévérité de l'échelle d'évaluation de symptômes du déficit de l'attention/hyperactivité AHD-RS (après 12 semaines de traitement, ainsi qu'une amélioration des scores de sévérité aux questionnaires de Conners parents (CPRS), le questionnaire de Conners enseignant (CTRS) et la CGI (impressions globales cliniques). La somnolence subjective est appréciée à l'aide de l'echelle ESEA (échelle de somnolence de l'enfant et de l'adolescent). La qualité de l'endormissement est appréciée au moyen de l'échelle de sévérité du syndrome des jambes sans repos.

Enfin la présente invention concerne également une composition pharmaceutique comprenant des excipients pharmaceutiquement acceptables, et du mazindol et comprenant en outre du fer ou un de ses sels pharmaceutiquement acceptable.

### Références

Biederman J, Swanson JM, Wigal SB, Kratochvil CJ, Boeflner SW, Earl CQ, Jiang J, Greenhill L.Efficacy and safety of modafinil film-coated tablets in children and adolescents with attention-deficit/hyperactivity disorder: results of a randomized, double-blind, placebo-controlled, flexible-dose study. Pediatrics 116: e777 2005
Biederman J, Heiligenstein JH, Fanes DE, Galil N, Dittmann R, Emslie GJ, Kratochvil CJ, Laws HF, Schuh KJ. Efficacy of atomoxetine versus placebo in school-age girls with attention-deficit/hyperactivity disorder. Pediatrics 110(6): 75 ; 2002
Busby K, Firestone P, Pivik RT - Sleep pattern in hyperkinetic and normal children. Sleep, 4, 366-83; 1981
Carskadon MA, Dement WC - Sleepiness in the normal adolescent. In: Sleep and its disorders in children. New York, Raven; 1987
Carskadon MA, Dement WC - The multiple sleep latency test: what does R measure? Sleep, 5, S67-72; 1982
Carskadon MA, Dement WC, Mitler MM, Roth T, Westbrook PR, Keenan S - Guidelines for the Multiple Sleep Latency Test (MSLT): a standard measure of sleepiness. Sleep 9:519-24; 1986
Chervin RD, Archbold KH, Dillon JE, Pituch KJ, Panahi P, DahI RE, Guilleminault C. Associations between symptoms of inattention, hyperactivity, restless legs, and periodic leg movements. Sleep 15;25(2):213-8; 2002.
Corkum P, Moldofsky H, Hogg-Johnson, Humphries T., Tannock R - Sleep problems in children with attention-deficit/hyperactivity disorder: impact of Subtype, comorbidity, and stimulant medication. J Am Acad Child Adolesc Psychiatry 38, 1285-93; 1999
Corkum P, Tannock R, Moldofsky H - Sleep disturbances in children with attention deficit hyperactivity disorder. J Am Acad Child Adolesc Psychiatry 37, 6, 637-46; 1998
Cortese S, Konofal E, Lecendreux M, Arnulf I, Mouren MC, Darra F, Dalla Bernardina B. Restless legs syndrome and attention-deficit/hyperactivity disorder: a review of the literature. Sleep. 2005 ;28(8):1007-13.
Golan N, Shahar E, Ravid S, Pillar G.Sleep disorders and daytime sleepiness in children with attention-deficit/hyperactive disorder. Sleep. 15;27:261-6; 2004
Greenhill LL, Puig-Antich J, Goetz R, Hanlon C - Sleep architecture and REM sleep measure in prepubertal children with attention deficit disorder with hyperactivity. Sleep 6,91-101; 1983
Hadier AJ. Mazindol, a new non-amphetamine anorexigenic agent. J Clin Pharmacol New Drugs. 12:453-8. 1972
Kaplan BJ, McNicol J, Conte RA, Moghadam HK. Sleep disturbance in preschool aged hyperactive and nonhyperactive children. Pediatrics 80: 839-44; 1987
Konofal E, Lecendreux M, Bouvard M and Mouren-Siméoni M-C - High levels of nocturnal activity in children with ADHD : a video analysis. Psychiatry Clin Neurosci 55,2,97-103; 2001
Konofal E, Lecendreux M, Mouren-Simeoni M-C. Sleep in children with attention deficit/hyperactivity disorder: a restatement on sleep studies. Ann Med Psychol 160: 105-17; 2002
Konofal E, Lecendreux M, Arnulf I, Mouren MC. Iron deficiency in children with attention-deficit/hyperactivity disorder. Arch Pediatr Adolesc Med. 2004 ;158(12):1 113-5.
Konofal E, Cortese S, Lecendreux M, Arnulf I, Mouren MC. Effectiveness of Iron supplementation in a young child With Attention-Deficit/Hyperactivity Disorder. Pediatrics 2005; 116 (5).
Konofal E, Cortese S. Restless legs syndrome and attention-deficit!hyperactivity disorder.
Ann Neurol. 2005 ;58(2):341-2
Lecendreux M, Konofal E, Bouvard M, Falissard B, Mouren-Simeoni M-C - Sleep and alertness in children with ADHD. J Child Psychol Psychiatry 41, 6, 803-12 ; 2000
Mick E, Biederman J, Jetton J, Faraone SV. Sleep disturbances associated with attention deficit hyperactivity disorder: the impact of psychiatric comorbidity and pharmacotherapy. J Child Adolesc Psychopharmacol FalI 10, 3:223-31; 2000
Palm L, Persson E, Bjerre L, Elmqvist D - Sleep and wakefulness in preadolescent children with deficits in attention, motor control and perception. Acta Paediatr 81, 618-24; 1992
Picchietti DL, Walters AS - Restless legs syndrome and periodic Iimb movement disorder in children and adolescents: comorbidity with attention-deficit/hyperactivity disorder. Child Adolesc. Psychiatry Clin N Am 5, 729-40; 1996
Platon MJR, Vela Bueno A, Espinar Sierra J, Kales S - Hypnopolygraphic alterations in attention deficit disorder (ADD) children. Intern J Neurosci 53, 87-101; 1990
Popper CW - Pharmacologic alternatives to psychostimulants for the treatment of attention-deficit/hyperactivity disorder. Child Adolesc Psychiatr Clin N Am 9, 3, 605-46 ; 2000
Rugino TA, Copley TC. Effects of modafinil in children with attention deficit/hyperactivity disorder: an open study. J Am Acad Child Adolesc Psychiatry 40(2) :230-5 ; 2001
Shindler J, Schachter M, Brincat S, Parkes JD. Amphetamine, mazindol, and fencamfamin in narcolepsy.Br Med J.20; 1167-70; 1985
Trommer BL, Hoeppner JB, Rosenberg RS, Armstrong KJ, Rothstein JA. Sleep disturbance in children with attention deficit disorder. Ann Neurology 24: 322 ; 1988
Walters AS, Mandelbaum DE, Lewin DS, Kugler S, England SJ, Miller M - Dopamincrgic thcrapy in children with restless legs/periodic limb movcmcnts in sleep and ADHD. Dopaminergic Therapy Study Group. Pediatr Neurol 22, 3, 182-6 ; 2000
Weinberg WA, Brumback RA - Primary disorder of vigilance: a novel explanation of inattentiveness, daydreaming, boredom, restlessness, and sleepiness. J Pediatr 116, 720-5; 1992
Weinberg WA, Harper CR - Vigilance and its disorders. Neu roI Clin 11, 59-78; 1993 Weiss M, Murray C, Weiss G. Adults with attention-deficit/hyperactivity disorder: current concepts. J Psychiatr Pract. 28, 99-111; 2002

## Revendications

1. Utilisation du mazindol pour la préparation d'un médicament destiné au traitement préventif et/ou curatif du trouble du déficit de l'attention/hyperactivité (TDAH) selon les critères du Manuel Diagnostique et statistique des troubles mentaux, 4^{ème} édition (DSM IV), chez un patient nécessitant un tel traitement, **caractérisée en ce que** la posologie correspond à une prise journalière de mazindol comprise entre 1 et 2 mg.

2. Utilisation selon la revendication 1 **caractérisée en ce que** ledit patient est choisi parmi un nouveau-né, un enfant, un adolescent, un adulte.

3. Utilisation selon la revendication 2, **caractérisée en ce que** ledit patient est un enfant présentant une carence martiale isolée mais non anémié.

4. Utilisation selon l'une quelconque des revendications 1 à 3 **caractérisée en ce que** le médicament est formulé pour permettre l'administration du mazindol par voie orale, anale, parentérale, intra-musculaire ou intraveineuse.

5. Utilisation selon l'une quelconque des revendications 1 à 4 en association avec du fer comme produit de combinaison pour une utilisation simultanée, séparée ou étalée dans le temps.

6. Utilisation selon l'une quelconque des revendications 1 à 5 en association avec au moins un composé sélectionné parmi les psycho-stimulants, notamment les inhibiteurs de la recapture de la dopamine et/ou de la noradrénaline comme produit de combinaison pour une utilisation simultanée, séparée ou étalée dans le temps.

7. Utilisation selon la revendication 6 **caractérisée en ce que** le dit composé est choisi parmi le methylphénidate, le modafinil, l'atomoxétine, et les amphétamines, notamment la d-amphétamine, la déxédrine, la dexamphétamine, la L-Dopa, la dopamine, les agonistes de la dopamine.

8. Utilisation selon l'une quelconque des revendications 1 à 7 **caractérisée en ce que** le dit patient est affecté d'une carence en ferritine, la dite concentration sérique en ferritine du dit patient étant inférieure à 50 µg/litre, inférieure à environ 40 µg/l, inférieure à environ 35 µg/l, inférieure à environ 30 µg/l, inférieure à environ 20 µg/l, à environ 15 µg/l, inférieure à environ 10 µg/l, inférieure à environ 5 µg/l.

9. Utilisation selon la revendication 8 **caractérisée en ce que** le dit patient présente en outre une concentration sérique normale de récepteurs solubles à la transferrine.

10. Utilisation selon l'une quelconque des revendications précédentes pour le traitement préventif de patient nouveau-né, adolescent, jeune adulte amené à développer à l'âge adulte une pathologie neuro-dégénérative **caractérisée en ce que** le dit patient nouveau-né, adolescent, jeune adulte présente au moins un des symptômes suivants :
- une carence en ferritine, de sorte que la concentration sérique en ferritine est inférieure à 50 µg/l,
- une concentration sérique normale des récepteurs solubles à transferrine
- un trouble du déficit de l'attention/hyperactivité, selon les critères du Manuel Diagnostique et statistique des troubles mentaux, 4^{ème} édition (DSM IV).

11. Utilisation selon la revendication 10 **caractérisée en ce que** la dite pathologie neurodégénérative est la maladie de Parkinson.

12. Composition pharmaceutique comprenant des excipients pharmaceutiquement acceptables et du mazindol, et comprenant en outre du fer ou un de ses sels pharmaceutiquement acceptable en tant que principes actifs.

## Patentansprüche

1. Verwendung von Mazindol für die Herstellung eines Medikaments, das zur vorbeugenden und/oder heilenden Behandlung der Aufmerksamkeitsdefizit/Hyperaktivitätsstörung (ADHS) nach den Kriterien des diagnostischen und statistischen Manuals mentaler Störungen, 4. Ausgabe (DSM IV), bei einem Patienten mit Bedarf an einer solchen Behandlung bestimmt ist, **dadurch gekennzeichnet, dass** die Dosierung einer täglichen Einnahme von Mazindol zwischen 1 und 2 mg entspricht.

2. Verwendung nach Anspruch 1, **dadurch gekennzeichnet, dass** der Patient ausgewählt ist aus einem Neugeborenen, einem Kind, einem Jugendlichen, einem Erwachsenen.

3. Verwendung nach Anspruch 2, **dadurch gekennzeichnet, dass** der Patient ein Kind ist, das einen isolierten Eisenmangel, jedoch keine Anämie aufweist.

4. Verwendung nach einem der Ansprüche 1 bis 3, **dadurch gekennzeichnet, dass** das Medikament so formuliert ist, dass es die Verabreichung von Mazindol auf oralem, analem, parenteralem, intramuskulärem oder intravenösem Wege gestattet.

5. Verwendung nach einem der Ansprüche 1 bis 4 in Verbindung mit Eisen als Kombinationspräparat für eine gleichzeitige, separate oder zeitlich gestaffelte Anwendung.

6. Verwendung nach einem der Ansprüche 1 bis 5 in Verbindung mit zumindest einer Verbindung ausgewählt aus Psychostimulanzien, insbesondere Dopamin- und/oder Noradrenalin-Wiederaufnahme-Inhibitoren als Kombinationspräparat für eine gleichzeitige, separate oder zeitlich gestaffelte Anwendung.

7. Verwendung nach Anspruch 6, **dadurch gekennzeichnet, dass** die Verbindung ausgewählt ist aus Methylphenidat, Modafinil, Atomoxetin, Amphetamine, insbesondere d-Amphetamin, Dexedrin, Dexamphetamin, L-Dopa, Dopamin, Dopamin-Agonisten.

8. Verwendung nach einem der Ansprüche 1 bis 7, **dadurch gekennzeichnet, dass** der Patient unter einem Ferritin-Mangel leidet, wobei die Ferritin-Serumkonzentration des Patienten unter 50 µg/Liter, unter etwa 40 µg/l, unter etwa 35 µg/l, unter etwa 30 µg/l, unter etwa 20 µg/l, unter etwa 15 µg/l, unter etwa 10 µg/l, unter etwa 5 µg/l, liegt.

9. Verwendung nach Anspruch 8, **dadurch gekennzeichnet, dass** der Patient ferner eine normale Serumkonzentration an löslichen Transferrin-Rezeptoren aufweist.

10. Verwendung nach einem der vorangehenden Ansprüche zur vorbeugenden Behandlung eines Patienten als Neugeborener, Jugendlicher, junger Erwachsener, bei dem naheliegt, im Erwachsenenalter eine neurodegenerative Erkrankung zu entwickeln, **dadurch gekennzeichnet, dass** der Patient als Neugeborener, Jugendlicher, junger Erwachsener zumindest eines der nachfolgenden Symptome aufweist:
- ein Ferritin-Mangel, so dass die Ferritin-Serumkonzentration unter 50 µg/l liegt,
- eine normale Serumkonzentration an löslichen Transferrin-Rezeptoren,
- eine Aufmerksamkeitsdefizit/Hyperaktivitätsstörung nach den Kriterien des diagnostischen und statistischen Manuals mentaler Störungen, 4. Ausgabe (DSM IV).

11. Verwendung nach Anspruch 10, **dadurch gekennzeichnet, dass** die neurodegenerative Erkrankung die Parkinson-Krankheit ist.

12. Pharmazeutische Zusammensetzung, enthaltend pharmazeutisch verträgliche Trägerstoffe und Mazindol und ferner enthaltend Eisen oder eines seiner pharmazeutisch verträglichen Salze als Wirkstoffe.

## Claims

1. Use of mazindol for preparing a medication intended for the preventive and/or curative treatment of attention-deficit/hyperactivity disorder (ADHD) according to the criteria of the Diagnostic and Statistical Manual of Mental Disorders, 4^{th} Edition (DSM IV), in a patient in need of such treatment, **characterised in that** the dosage consists of a daily dose of mazindol between 1 and 2 mg.

2. Use according to claim 1, **characterised in that** said patient is chosen from a newborn baby, a child, an adolescent or an adult.

3. Use according to claim 2, **characterised in that** said patient is a child having isolated iron deficiency, but not anaemic.

4. Use according to any one of claims 1 to 3, **characterised in that** the medication is formulated to allow the administration of mazindol by oral, anal, parenteral, intra-muscular or intravenous route.

5. Use according to any one of claims 1 to 4, in combination with iron as a combination product for simultaneous, separate or sequential use.

6. Use according to any one of claims 1 to 5, in combination with at least one compound selected from psychostimulants, in particular dopamine and/or noradrenaline reuptake inhibitors, as a combination product for simultaneous, separate or sequential use.

7. Use according to claim 6, **characterised in that** said compound is chosen from methylphenidate, modafinil, atomoxetine, amphetamines, in particular d-amphetamine, dexedrine, dexamphetamine, L-Dopa, dopamine, and dopamine agonists.

8. Use according to any one of claims 1 to 7, **characterised in that** said patient suffers from ferritin deficiency, said serum ferritin concentration of said patient being less than 50 µg/litre, less than approximately 40 µg/l, less than approximately 35 µg/l, less than approximately 30 µg/l, less than approximately 20 µg/l, less than approximately 15 µg/l, less than approximately 10 µg/l or less than approximately 5 µg/l.

9. Use according to claim 8, **characterised in that** said patient also has a normal serum concentration of transferrin soluble receptors.

10. Use according to any one of the preceding claims for the preventive treatment of a newborn, adolescent or young adult patient liable to develop a neurodegenerative pathology at an adult age, **characterised in that** said newborn, adolescent or young adult patient has at least one of the following symptoms:
- a ferritin deficiency, so that the serum ferritin concentration is less than 50 µg/l,
- a normal serum concentration of transferrin soluble receptors,
- attention-deficit/hyperactivity disorder, according to the criteria of the Diagnostic and Statistical Manual of Mental Disorders, 4^{th} Edition (DSM IV).

11. Use according to claim 10, **characterised in that** said neurodegenerative pathology is Parkinson's disease.

12. Pharmaceutical composition comprising pharmaceutically acceptable excipients and mazindol, and further comprising iron or any of the pharmaceutically acceptable salts thereof as active substances.
